**Europäisches Patentamt**

**European Patent Office**

**Office européen des brevets**

(19)

(11) Veröffentlichungsnummer : **0 431 477 B1**

(12) **EUROPÄISCHE PATENTSCHRIFT**

(45) Veröffentlichungstag der Patentschrift :
26.01.94 Patentblatt 94/04

(51) Int. Cl.⁵ : **C07C 69/82, C07C 67/52**

(21) Anmeldenummer : **90122903.9**

(22) Anmeldetag : **30.11.90**

(30) Priorität : **02.12.89 DE 3939913**
**30.05.90 DE 4017343**

(43) Veröffentlichungstag der Anmeldung :
**12.06.91 Patentblatt 91/24**

(45) Bekanntmachung des Hinweises auf die
Patenterteilung :
**26.01.94 Patentblatt 94/04**

(84) Benannte Vertragsstaaten :
**BE DE DK ES FR GB GR IT LU NL**

(56) Entgegenhaltungen :
**WO-A-90/09367**
**US-A- 3 845 100**

(73) Patentinhaber : **HOECHST**
**AKTIENGESELLSCHAFT**
**D-65926 Frankfurt (DE)**

(72) Erfinder : **Bachmann, Wilfried**
**Am Trammer See 13**
**W-2320 Plön (DE)**
Erfinder : **Bader, Rolf, Dr.**
**Pestalozzistrasse 39**
**W-6057 Dietzenbach (DE)**
Erfinder : **Büttner, Horst, Dr.**
**Kronthaler Strasse 15**
**W-6232 Bad Soden am Taunus (DE)**
Erfinder : **Wetzel, Edgar, Dr.**
**Breslauer Strasse 60**
**W-6056 Heusenstamm (DE)**

(54) **Verfahren zum Reinigen von Dimethylterephthalat.**

Jouve, 18, rue Saint-Denis, 75001 PARIS

**Beschreibung**

Die Erfindung betrifft ein Verfahren zum Reinigen von Dimethylterephthalat (DMT) durch Umkristallisation aus methanolischer Lösung, bei dem man die Mutterlauge, die die Verunreinigungen enthält, abtrennt und einer Aufarbeitung zuführt und das feuchte Kristallisat durch Schmelzen und Verdampfen des Methanols trocknet.

Bei der Herstellung von DMT durch katalytische Oxydation von p-Xylol mit Luft und anschließender Veresterung mit Methanol entstehen u.a. die Isomere Dimethylisophthalat (DMI) und Dimethylorthophthalat (DMO) sowie Aldehyde als Verunreinigungen, die abgetrennt werden müssen. Das geschieht im wesentlichen durch das eingangs beschriebene Verfahren. Dieses Verfahren hat jedoch den Nachteil, daß für die Reinigung etwa doppelt soviel Methanol bezogen auf den Gewichtsanteil des DMT im Roh-DMT, wie es aus der Rektifikation kommt, als Lösemittel eingesetzt werden muß. Ferner enthält der Feststoff aus der Methanolaufarbeitung vorwiegend DMT. Um Verluste zu vermeiden, muß er in das Verfahren rezirkuliert werden. Durch dieses Rezirkulieren steigt der Isomerenpegel (DMI, DMO) in der Anlage stetig an. Die Isomeren müssen daher von Zeit zu Zeit ausgeschleust werden, was jedoch nur mit einem beträchtlichen Verlust an DMT möglich ist. Hier will die Erfindung Abhilfe schaffen.

Die Erfindung löst die Aufgabe dadurch, daß die Mutterlauge durch Filtern abgetrennt und das aus der Abtrennung erhaltene feuchte Kristallisat vor dem Schmelzen gewaschen und die Waschflüssigkeit mit einem Teil der abgetrennten Mutterlauge in die Umkristallisation zurückgeführt wird. Das Kristallisat kann mit Methanol gewaschen werden.

In einer Ausgestaltung wird das gewaschene Kristallisat in einer zweiten Stufe aus Methanol umkristallisiert, die Mutterlauge abgetrennt, einen Teil der Mutterlauge in die Umkristallisation der zweiten Stufe und den anderen Teil in die Umkristallisation der ersten Stufe als Lösungsmittel sowie der Kristallwäsche der ersten Stufe als Waschflüssigkeit zugeführt.

Das Kristallisat aus der zweiten Stufe der Umkristallisation kann nach dem Abtrennen der Mutterlauge mit Methanol gewaschen und die Waschflüssigkeit in die Umkristallisation der zweiten Stufe zugeführt werden. Das Kristallisat aus der Umkristallisation der ersten Stufe kann mit Mutterlauge aus der Abtrennung nach der Umkristallisation der zweiten Stufe gewaschen werden.

Der Vorteil des erfindungsgemäßen Verfahrens liegt darin, daß für die Reinigung von DMT erheblich weniger Methanol - etwa die Hälfte wie nach dem bekannten Verfahren - bei gleichbleibender bzw. verbesserter DMT-Qualität benötigt wird.

Im folgenden wird die Erfindung anhand von Figuren näher erläutert. Es zeigt

Figur 1      das Fließschema für ein einstufiges Verfahren;
Figur 2      das Fließschema für ein zweistufiges Verfahren;
Figur 3      das Fließschema für eine Alternative zum zweistufigen Verfahren gemäß Figur 2 und
Figur 4      das Fließschema für ein zweistufiges Verfahren gemäß dem Stand der Technik.

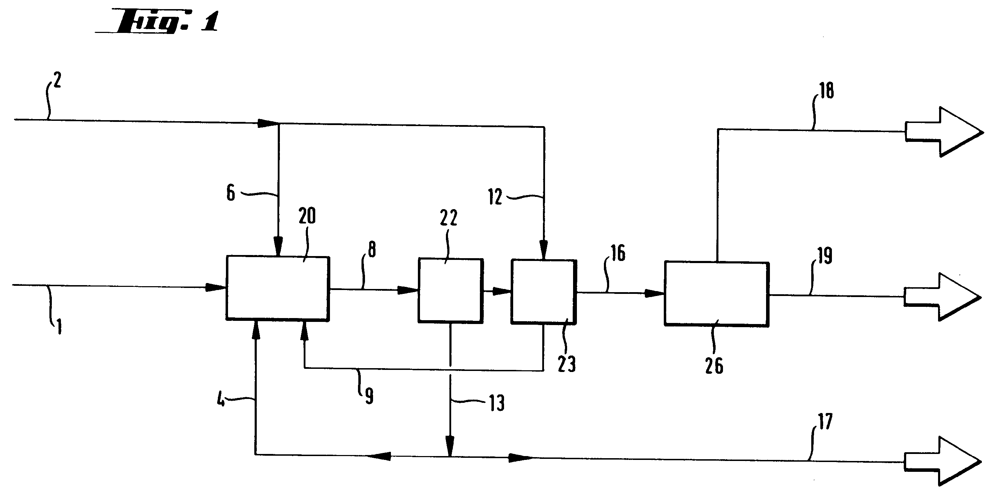

Nach Figur 1 wird das die Verunreinigungen DMI, DMO und Aldehyde enthaltende DMT (Roh-DMT) über Leitung (1) der Umkristallisierung (20) zugeführt und über Leitung 6 ein Teil des erforderlichen Lösemittels Methanol. Die in (20) anfallende Kristallsuspension gelangt über Leitung (8) in die Abtrennung (22), auf einem Filter (Fritte, (Druck)nutsche, Bandfilter etc.) wo die Mutterlauge abgetrennt wird. Ein Teil der Mutterlauge Leitung (13) wird über Leitung (4) der Umkristallisierung (20) und der andere Teil der Mutterlauge über Leitung (17) der Aufbereitung (nicht dargestellt) zugeführt. Das aus der Abtrennung (22) kommende Kristallisat wird in (23) mit Methanol gewaschen, das über Leitung (12) zugeführt und vorzugsweise in Form einer Pfropfenströmung durch das Kristallisat geleitet wird. Die aus der Wäsche (23) kommende Waschflüssigkeit wird über Leitung (9) ebenfalls in die Umkristallisierung (20) zurückgeführt. Das aus der Wäsche (23) kommende feuchte Kristallisat gelangt über Leitung (16) in den Schmelzer (26) von wo über Leitung (18) der Methanoldampf und über Leitung (19) das gereinigte DMT abgezogen wird.

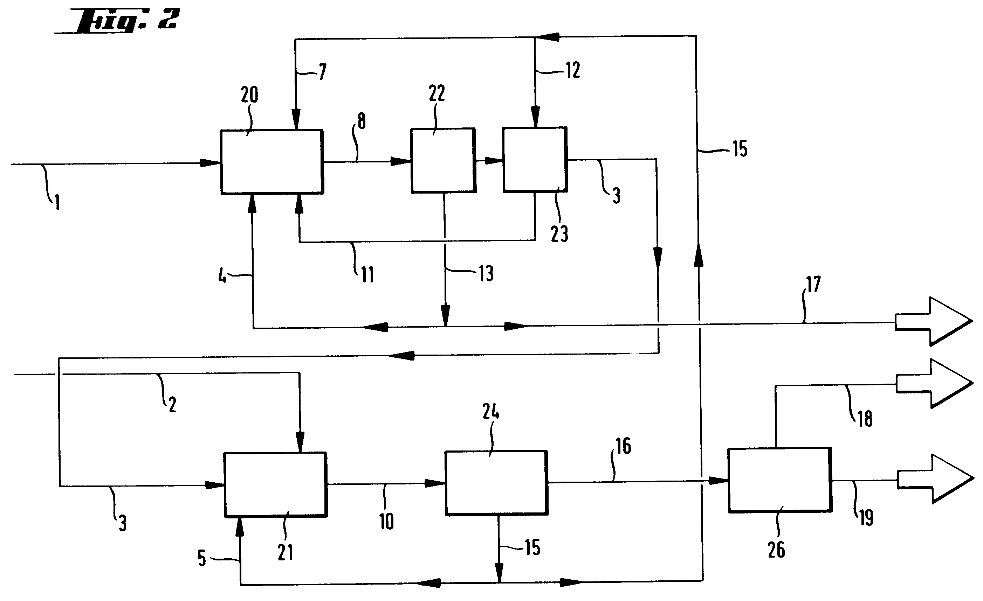

Gemäß Figur 2 wird das aus Wäsche (23) kommende Kristallisat (Leitung 3) in einer zweiten Stufe (21) in Methanol umkristallisiert. Die Kristallsuspension der 2. Stufe 21 wird über Leitung 10 der Abtrennung 24 zugeführt. Das Methanol wird der zweiten Umkristallisation (21) über Leitung (2) zugeführt. Ein Teil dar in der Abtrennung (24), einem Filter, abgetrennten Mutterlauge wird über Leitungen (15) und (5) in die zweite Stufe (21) der Umkristallisierung zurückgeführt und der andere Teil über Leitungen (15) und (12) der Wäsche (23) und über Leitungen (15) und (7) der Umkristallisierung (20) zugeführt. Das aus der Abtrennung (24) resultierende Kristallisat gelangt über Leitung (16) in den Schmelzer (26) aus dem über Leitung (18) Methanoldampf und über Leitung (19) reines DMT abgezogen wird. Das über Leitung (1) in die erste Umkristallisierung (20) aufgegebene verunreinigte DMT (Roh-DMT) wird im wesentlichen in Mutterlauge gelöst, die aus der Abtrennung (24) der zweiten Stufe kommt. Von der aus der Abtrennung (22) kommenden Mutterlauge, die am stärksten mit Verunreinigungen belastet ist, wird ein Teil über Leitungen (13) und (17) der Mutterlaugeaufbereitung (nicht dargestellt) zugeführt, während der andere Teil über Leitungen (13) und (4) in die Umkristallisierung (20) rezirkuliert wird. Die Waschflüssigkeit aus der der Abtrennung (22) folgenden Wäsche (23) wird über Leitung (11) der Umkristallisierung (20) zugeführt.

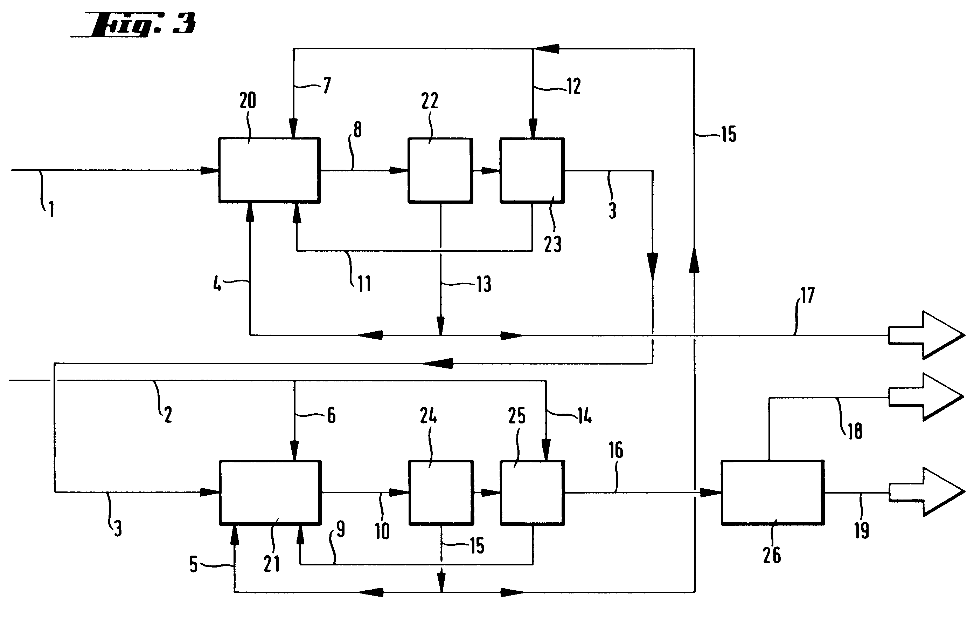

Die Schaltung gemäß Figur 3 entspricht weitgehend der Schaltung gemäß Figur 2 mit dem Unterschied, daß der Abtrennung (24) eine Kristallwäsche (25) mit Methanol nachgeschaltet ist, das aus den Leitungen (2) und (14) kommt. Die Waschflüssigkeit wird über Leitung (9) der zweiten Umkristallisation (21) zugeführt.

Die Schaltung gemäß Figur (4) entspricht dem Stand der Technik. Das mit DMI, DMO und Aldeyhde verunreinigte DMT wird der Umkristallisierung (20) über Leitung (1) zugeführt und über (7) das für die Umkristallisation erforderliche Lösemittel. Die in der Umkristallisation (20) anfallende Kristallsuspension wird über Leitung (8) einer Zentrifuge (22a) zugeführt, wo die Mutterlauge abgetrennt wird, die über Leitung (17) in die Aufarbeitung (nicht dargestellt) gelangt. Das Kristallisat (Leitung 3) wird in einer zweiten Stufe (21) in Methanol, das dieser Stufe über Leitung (2) zugeführt wird, umkristallisiert und die Kristallsuspension (Leitung 10) in Zentrifuge (24a) von der Mutterlauge befreit. Das Kristallisat gelangt über Leitung (16) in den Schmelzer (26), aus dem über Leitung (18) der Methanoldampf und Leitung (19) reines DMT abgezogen wird. Die in Zentrifuge (24a) abgetrennte Mutterlauge wird zum Lösen des Roh-DMT über Leitung (7) in die Umkristallisation (20) geleitet.

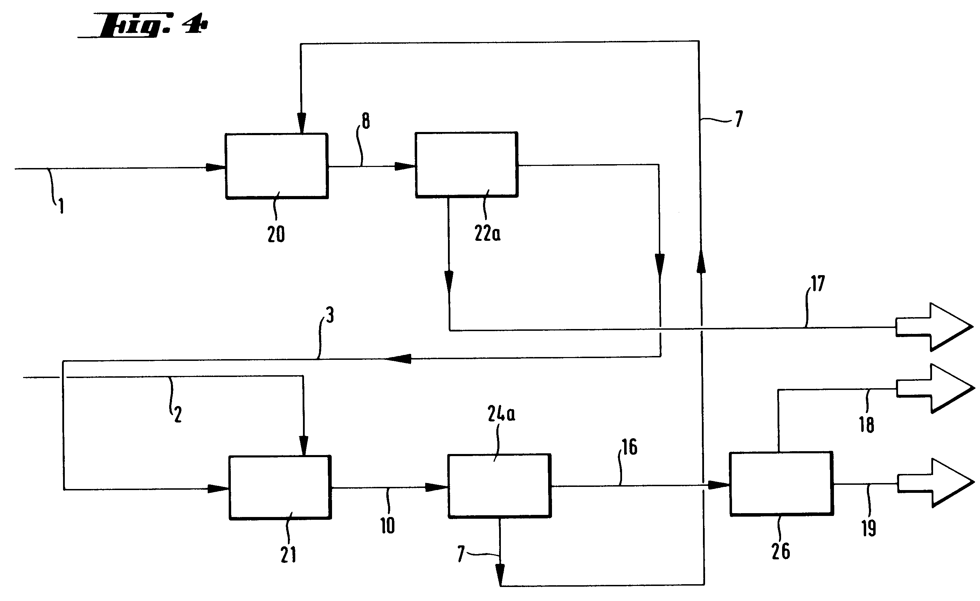

In der Tabelle sind die Strombilanzen der einzelnen Schaltungen gegenübergestellt. Die Nummern 1 bis 4 entsprechenen den Schaltungen gemäß Figuren 1 bis 4. Aus der Tabelle ist ersichtlich, daß bei halbem Methanolbedarf ein weniger verunreinigtes Rein-DMT erhalten wird. Selbst bei dem einstufigen Verfahren (Figur 1) liegen die Verunreinigungen in der gleichen Größenordnung wie beim bekannten zweistufigen Verfahren (Figur 4), das doppelt soviel Methanol für die Reinigung benötigt. Ferner geht aus der letzten Spalte der Tabelle hervor, daß die Verfahren gemäß Fig. 1, 2 und 3 mit Mutterlaugen betrieben werden können, die einen wesentlich höheren Anteil an Verunreinigungen (Isomeren) aufweisen als die Mutterlauge, die zum Betrieb des Verfahrens gemäß Fig. 4 erforderlich ist.

Tabelle

| Schaltg gemäß Figur | Nr. der Lei- tung | Bezeichnung | Roh- DMT [t] | Rein-DMT [t] | Methanol [t] | Verun- reinigungen insgesamt [t] |
|---|---|---|---|---|---|---|
| 1 | 1 | Roh-DMT | 109,3 | 100 | - | 9,3 |
| 2 | 1 | Roh-DMT | 115,8 | 100 | - | 15,8 |
| 3 | 1 | Roh-DMT | 112,0 | 100 | - | 12,0 |
| 4 | 1 | Roh-DMT | 115,8 | 100 | - | 15,8 |
| 1 | 2 | Rein-Methanol | 90 | - | 90 | - |
| 2 | 2 | Rein-Methanol | 90 | - | 90 | - |
| 3 | 2 | Rein-Methanol | 90 | - | 90 | - |
| 4 | 2 | Rein-Methanol | 180 | - | 180 | - |
| 1 | 17 | Mutterlauge zur Aufarbeitung | 67,5 | 1,1 | 57,0 | 9,3 |
| 2 | 17 | Mutterlauge zur Aufarbeitung | 102,4 | 1,7 | 84,8 | 15,8 |
| 3 | 17 | Mutterlauge zur Aufarbeitung | 71,1 | 1,8 | 57,3 | 12,0 |
| 4 | 17 | Mutterlauge zur Aufarbeitung | 193,6 | 3 | 174,8 | 15,8 |
| 1 | 18 | Methanoldampf aus Schmelzer | 33,0 | - | 33,0 | - |
| 2 | 18 | Methanoldampf aus Schmelzer | 5,2 | - | 5,2 | - |
| 3 | 18 | Methanoldampf aus Schmelzer | 32,7 | - | 32,7 | - |
| 4 | 18 | Methanoldampf aus Schmelzer | 5,2 | - | 5,2 | - |
| 1 | 19 | Rein-DMT | 98,9 | 98,9 | - | 0,0450 |
| 2 | 19 | Rein-DMT | 98,3 | 98,3 | - | 0,0018 |
| 3 | 19 | Rein-DMT | 98,2 | 98,2 | - | 0,0001 |
| 4 | 19 | Rein-DMT | 97 | 97 | - | 0,02 |

**Patentansprüche**

1. Verfahren zum Reinigen von Dimethylterephthalat (DMT) durch Umkristallisation aus methanolischer Lösung, bei dem man die Mutterlauge, die die Verunreinigungen enthält, abtrennt und einer Aufbereitung zuführt und das feuchte Kristallisat durch Schmelzen und Verdampfen des Methanols trocknet, dadurch gekennzeichnet, daß die Mutterlauge durch Filtern abgetrennt und das aus der Abtrennung erhaltene feuchte Kristallisat vor dem Schmelzen gewaschen und die Waschflüssigkeit mit einem Teil der abgetrennten Mutterlauge in die Umkristallisation zurückgeführt wird.

2. Verfahren nach Anspruch 1, dadurch gekennzeichnet, daß das Kristallisat mit Methanol gewaschen wird.

3. Verfahren nach Anspruch 1, dadurch gekennzeichnet, daß man das gewaschene Kristallisat in einer zweiten Stufe aus Methanol umkristallisiert, die Mutterlauge abtrennt, einen Teil in die Umkristallisation der zweiten Stufe und den anderen Teil in die Umkristallisation der ersten Stufe als Lösemittel sowie der Kristallwäsche der ersten Stufe als Waschflüssigkeit zuführt.

4. Verfahren nach Anspruch 3, dadurch gekennzeichnet, daß man das Kristallisat aus der zweiten Stufe der Umkristallisation nach dem Abtrennen der Mutterlauge mit Methanol wäscht und die Waschflüssigkeit in die Umkristallisation der zweiten Stufe führt.

5. Verfahren nach Anspruch 3 oder 4, dadurch gekennzeichnet, daß das Kristallisat aus der Umkristallisation der ersten Stufe mit Mutterlauge aus der Abtrennung nach der zweiten Umkristallisation wäscht.

**Claims**

1. A process for the purification of dimethyl terephthalate (DMT) by recrystallization from methanolic solution, in which the mother liquor containing the impurities is separated off and passed to a workup tank, and the moist crystals are dried by melting and evaporating the methanol, which comprises washing the moist crystals obtained from the separation before melting and recycling the washing liquid together with a portion of the separated mother liquor into the recrystallization tank.

2. The process as claimed in claim 1, wherein the crystals are washed with methanol.

3. The process as claimed in claim 1, wherein the washed crystals are recrystallized in a second step from methanol, the mother liquor is separated off, a portion is used for the recrystallization in the second step and the other portion for the recrystallization in the first step as the solvent and used as the washing liquid for washing the crystals from the first step.

4. The process as claimed in claim 3, wherein the crystals from the second recrystallization step are washed with methanol, after the mother liquor has been separated off, and the washing liquid is passed to the recrystallization tank of the second step.

5. The process as claimed in claim 3 or 4, wherein the crystals from the recrystallization in the first step are washed with mother liquor from the separation after the second recrystallization.

**Revendications**

1. Procédé de purification de téréphtalate de diméthyle (DMT) par recristallisation dans une solution méthanolique, dans lequel on sépare la liqueur mère qui contient les impuretés et on l'envoie vers un traitement, et on sèche le produit cristallisé humide par fusion et évaporation du méthanol, caractérisé en ce que l'on sépare la liqueur mère à travers des filtres et on lave le produit cristallisé humide obtenu à partir de la séparation avant de le fondre, et on renvoie dans la recristallisation le liquide de lavage avec une partie de la liqueur mère séparée.

2. Procédé selon la revendication 1, caractérisé en ce que le produit cristallisé est lavé avec du méthanol.

3. Procédé selon la revendication 1, caractérisé en ce que dans une seconde étape on recristallise dans le méthanol le produit cristallisé lavé, on sépare la liqueur mère, on en renvoie une partie dans la recristallisation de la seconde étape et l'autre partie dans la recristallisation de la première étape en tant que solvant, ainsi que dans le lavage des cristaux de la première étape en tant que liquide de lavage.

4. Procédé selon la revendication 3, caractérisé en ce que l'on lave avec du méthanol le produit cristallisé issu de la seconde étape de recristallisation après la séparation de la liqueur mère, et on envoie le liquide de lavage dans la recristallisation de la seconde étape.

5. Procédé selon la revendication 3 ou 4, caractérisé en ce que l'on lave le produit cristallisé issu de la recristallisation de la première étape avec la liqueur mère issue de la séparation suivant la seconde recristallisation.

Fig. 1

2

18

6

12

20

22

16

8

1

19

23

4

9

13

26

17

EP 0 431 477 B1

# Fig. 2

EP 0 431 477 B1

Fig. 3

EP 0 431 477 B1

EP 0 431 477 B1

# Fig. 4